# EUROPÄISCHE PATENTSCHRIFT

(11) **EP 1 933 767 B1**
(45) Veröffentlichungstag und Bekanntmachung des Hinweises auf die Patenterteilung: **03.04.2013**
(21) Anmeldenummer: 06762255.5
(22) Anmeldetag: 29.06.2006
(51) Int. Cl.: A61K 35/16, A61K 38/18, A61K 38/22, A61F 2/08, A61L 27/36

(54) **ZELLFREIES TRANSPLANTAT AUS MATRIX UND SERUM**
CELL-FREE GRAFT CONSISTING OF A MATRIX AND A SERUM
GREFFON ACELLULAIRE FAIT D'UNE MATRICE ET DE SERUM

(30) Priorität: 30.06.2005 DE 102005030614
(43) Veröffentlichungstag der Anmeldung: 25.06.2008
(73) Patentinhaber: BioTissue AG, 8008 Zürich (CH)
(72) Erfinder: SITTINGER, Michael, 12305 Berlin (DE); TÁNCZOS, Eszter, 8700 Kuesnacht (DE); KAPS, Christian, 10965 Berlin (DE)
(74) Vertreter: Westendorp | Sommer
(86) Internationale Anmeldenummer: PCT/EP2006/006281
(87) Internationale Veröffentlichungsnummer: WO 2007/003324

(56) Entgegenhaltungen:
- WO-A-01/66130
- WO-A-02/38163
- DE-A1- 10 042 484
- GB-A- 1 052 589
- US-B1- 6 506 217

## Beschreibung

Die vorliegende Erfindung betrifft ein zellfreies Transplantat zur Gewebsregeneration und insbesondere zur Knorpelregeneration, ein Verfahren zu dessen Herstellung und die Verwendung des Transplantats zur Gewebsregeneration.

### Stand der Technik

Knorpel ist eine vom Bindegewebe abgeleitete mesodermale Gewebsform, welche auf multipotente, undifferenzierte mesenchymale Vorläuferzellen zurückzuführen ist. Man unterscheidet drei Arten von Knorpel, den hyalinen, den elastischen und den Faserknorpel. Der hyaline Knorpel ist die am weitesten verbreitete Knorpelform und findet sich beispielsweise in den Gelenkflächen. Knorpeldefekte aufgrund von Abnutzung oder Beschädigung stellen ein weit verbreitetes medizinisches Problem dar. Aufgrund der Abgeschlossenheit des Knorpels vom klassischen Entzündungs- und Reparatursystem des Körpers besteht nur eine geringe Fähigkeit zur Selbstheilung. Dementsprechend wurden schon in der Vergangenheit, vor allem aber in den letzten Jahren Methoden und Techniken entwickelt, um defekte chondrale oder auch osteochondrale Areale im Gelenkknorpel zu ersetzen. So wurden als Gelenkknorpelersatz Periost-, Perichondrium-, allogene und autologe osteochondrale Transplantate, allogene Menisken oder auch Prothesen aus künstlichen Materialien eingesetzt.

Bei der autologen Transplantation von Chondrozyten werden dem Patienten entnommene Chondrozyten in Zellkultur vermehrt und dem Patienten wieder zurückgegeben. Die Rückführung kann in Form verschiedenster Transplantate erfolgen. Beispiele hierfür sind Injektionslösungen, die in das Gelenk injiziert werden, mit Knorpelzellen beimpfte Matrices und Ähnliches.

Beispielsweise beschreibt die WO 97/15655 künstliche Gewebe, die aus dreidimensionalen extrazellulären Matrices und gentechnisch manipulierten Zellen bestehen, wobei die Matrices immunsuppressive oder zelldifferenzierende Faktoren freisetzen können. Bevorzugt handelt es sich um Matrices in Form eines Polymervlieses, in die eine Zellsuspension, die beispielsweise in einer Fibrinogenlösung suspendiert sein kann, verteilt wird. Der Matrix können zudem Faktoren oder Komponenten der entsprechenden extrazellulären Matrix zugesetzt werden, die dem Wachstums- und/oder Differenzierungsprozess förderlich sind. Um die Zellen in der Matrix zu halten, kann die Zellsuspension durch Thrombinzugabe verfestigt werden, um das fertige Transplantat zu erhalten.

Die DE 44 31 598 beschreibt ein Verfahren zum Herstellen eines Implantates aus Zellkulturen, bei dem dreidimensionale Trägerstrukturen, an die Zellen angelagert sind, zunächst ummantelt und anschließend mit einer Nährlösung perfundiert werden. In die Trägerstrukturen sind resorbierbare Mikrokörper eingearbeitet, die bei der Resorption die Gewebebildung beeinflussende Faktoren freigeben.

Die DE 43 06 661 beschreibt eine dreidimensionale Trägerstruktur, vorzugsweise aus einem Polymervlies, in die Zellen eingelagert werden. Die Trägerstruktur wird anschließend in Nährlösung perfundiert, um das Zellwachstum und die Ausbildung einer extrazellulären Matrix durch die Zellen zu fördern. Um ein Auswandern bzw. Ausspülen der Zellen zu verhindern, wird die Trägerstruktur mit Agarose ummantelt.

Aus der DE 101 39 783 ist ferner bekannt die Bereitstellung von mesenchymalen Zellen in Synovialflüssigkeit. Diese Zusammensetzung kann, falls gewünscht, auch auf einen Träger wie ein Vlies oder einen Kunststoff aufgebracht und in dieser Form als Transplantat verwendet werden. Ansonsten wird die Suspension der Zellen in Synovialflüssigkeit als solches in das betroffene Gelenk injiziert.

Alternativ werden Matrixstrukturen synthetisiert, welche selbst keine Zellen enthalten. So beschreibt beispielsweise die US 2003/0003153 versteifte Matrixmembranen, die ein oder mehrere gerüstbildende Proteine enthalten, welche für das Zellwachstum geeignet sind. Geeignete Proteine sind beispielsweise Kollagen. Die erhaltenen Matrices in Membranform können mit Zellen beimpft oder als solches transplantiert werden. In letzterem Falle wird davon ausgegangen, dass Zellen aus körpereigenem Gewebe in die Matrixstruktur einwandern. Dieses wird beispielsweise mittels der konventionellen Pridie-Bohrung oder Mikrofrakturierung angewandt. Bei diesen Techniken werden geringfügige Bohrungen oder Frakturen in den Gelenkknochen bis auf das Knochenmark eingebracht. Durch die Bohrungen kommt es zur Einblutung in den Defekt, wodurch sich der Defekt mit einem Blutpfropf füllt. In dem Pfropf befinden sich mesenchymale Vorläuferzellen, welche durch entsprechende Reize stimuliert ein knorpelartiges Ersatzgewebe, den sog. Faserknorpel bilden können. Wird über der Pridie-Bohrung ein Matrixmaterial bereitgestellt, können die Blutzellen in dieses Matrixmaterial einwandern und sich dort ansiedeln.

Die DE 199 57 388 und WO 2005/014027 nutzen diesen Effekt und verstärken ihn, indem in der Matrixstruktur Wachstums- und Differenzierungsfaktoren (DE 199 57 388) bzw. Chemokine (WO 2005/014027) als Rekrutierungsmittel bereitgestellt werden. Alle Faktoren sollen zur verstärkten Rekrutierung der knorpelbildenden mesenchymalen Vorläuferzellen führen, wodurch letztlich eine schnellere Regeneration des Knorpels erzielt werden soll.

Aus der WO 02/00272 ist schließlich die Möglichkeit bekannt, entsprechende Transplantate auch aus Blut und einer Polymerkomponente herzustellen. Diesem Dokument liegt das Problem zugrunde, dass der bei der Pridie-Bohrung üblicherweise entstehende Blutpfropf sich bei der Gerinnung zusammenzieht und damit die Form verändert. Das zugesetzte Polymer verhindert diese Formveränderung und gestattet somit eine formtreue Verheilung. Zur Herstellung des Transplantats wird ein Polymer mit Blut oder einer Blutkomponente wie Erythrocyten, Leukozyten, Monozyten, Plättchen, Fibrinogen, Thrombin und plättchenreichem Plasma gemischt und in den Defekt eingebracht. Bei der Verwendung einer Blutkomponente ist jedoch das Vorhandensein gerinnungsfähigen Materials wesentlicher Bestandteil, um die gewünschte Wirkung zu erzielen.

Alternativ können Transplantate aus Chitosan und Chondrozyten verwendet werden. Da hier die Zellen wie oben von vornherein in den Defekt eingebracht werden, kann auf den Zusatz von Lockstoffen und/oder Wachstums- und Differenzierungsfaktoren verzichtet werden.

GB 1,052,589 offenbart Gerüstmatrixmaterialien zur Regeneration von Blutgefässen, die aus einer Gel-Lösung hergestellt werden, wobei das resultierende Transplantat in einem weiteren Schritt mit natürlichem Blutserum imprägniert wird.

Bei den oben beschriebenen Technologien bestehen Nachteile dahingehend, dass dann, wenn das Transplantat selbst Zellen enthält, diese häufig durch die Manipulation bei der Handhabung beschädigt werden, das Transplantat bei Verwendung von Zellen, insbesondere autologer Zellen über ein langwieriges Kulturverfahren hergestellt werden muss und die sorgfältige Kontrolle auf Kontaminationen erfordert und schließlich keine Lagerfähigkeit besteht. Parallel hat sich die Rekrutierung bei zellfreien Transplantaten von mesenchymalen Zellen durch die Pridie-Bohrung hindurch mit oder ohne Lockstoff als unzureichend erwiesen. Die Besiedlung ist langsam, geht von wenigen Zellen aus und ist zudem unspezifisch. Dies heißt, dass durch das aus der Pridie-Bohrung austretende Blut verschiedene Zelltypen in das Transplantat eingespült werden und dort verbleiben. Gewünscht wird jedoch lediglich die Besiedlung durch sich zu Chondrozyten differenzierende mesenchymale Vorläuferzellen. Dieses ist bei den herkömmlichen Transplantaten jedoch nicht gewährleistet.

Die vorliegende Erfindung hat daher unter Anderem die Aufgabe, ein Transplantat bereitzustellen, welches einfach herzustellen, gut zu lagern und einfach in der Anwendung ist. Des Weiteren wäre es wünschenswert, die Rekrutierungsraten durch das Transplantat zu erhöhen, eine bessere Selektivität für die Art der rekrutierten bzw. im Transplantat befindlichen Zellen zu erzielen und möglichst auf die Verwendung von körperfremden, ggf. so gar rekombinanten Wachsumsfaktoren, welche potentiell Allergene darstellen, verzichten zu können.

### Zusammenfassung der Erfindung

Die vorliegende Erfindung löst diese und andere Probleme des Standes der Technik. Hierzu stellt sie ein zellfreies Transplantat gemäß Anspruch 1 bereit, umfassend (i) eine zusammenhängende, gerüstbildende Matrix mit offener Porosität aus einem biologisch und pharmazeutisch annehmbaren Material und (ii) Blut-Serum. Die Matrix enthält zusätzlich ein Gel.

In einem zweiten Aspekt wird ein Verfahren zur Herstellung eines solchen zellfreien Transplantats bereitgestellt, bei dem man die Matrix und das Gel, mit dem Serum in Kontakt bringt. Ggf. kann das Transplantat mit Serum getrocknet werden. Alternativ können die Matrix und das Gel vor In-Kontakt-Bringen mit dem Serum in getrocknetem Zustand vorliegen.

In einem dritten Aspekt stellt die vorliegende Erfindung schließlich die Verwendung des zellfreien Transplantats zur Gewebsregeneration und insbesondere zur Regeneration von Knorpel und/oder Knochen bereit.

### Kurze Beschreibung der Zeichnungen

Fig. 1 zeigt die chemotaktische Wirkung von CDMP1, CDMP2, SDF1-α und IL8 auf humane mesenchymale Stammzellen in vitro;
Fig. 2 zeigt die chemotaktische Wirkung von humanem Serum des Blutes auf humane mesenchymale Stammzellen in vitro.

### Genaue Beschreibung der Erfindung

Die vorliegende Erfindung betrifft, wie oben beschrieben, ein zellfreies Transplantat, gemäß Anspruch 1 umfassend (i) eine zusammenhängende, gerüstbildende Matrix mit offener Porosität aus einem biologisch und pharmazeutisch annehmbaren Material und (ii) Blut-Serum. Die Verwendung von Serum im erfindungsgemäßen zellfreien Transplantat gestattet überraschenderweise eine Erhöhung der Rekrutierungseffizienz von mesenchymalen Vorläuferzellen aus dem durchbluteten Knochenmark um mehrere Größenordnungen. Diese überrascht gesteigerte Rekrutierungseffizienz erlaubt es wiederum, auf die separate Einbringung von ausdifferenzierten Zellen oder Vorläuferzellen im Transplantat selbst zu verzichten, was die Handhabung erleichtert, die Herstellung des Transplantats vereinfacht und abkürzt und auch eine Lagerung ermöglicht.

Blutserum ist auf herkömmlichem Wege in einfacher Weise zu gewinnen. Dieses kann vorzugsweise direkt während der Transplantation vom Patienten selbst erfolgen. Dem Patienten kann damit autologes Material re-implantiert werden, während ein Zusatz von anderen potentiell allergenen und/oder immunaktiven Faktoren nicht erforderlich ist.

Die Matrix des erfindungsgemäßen zellfreien Transplantats ist eine zusammenhängende, gerüstbildende Matrix mit offener Porosität. Mit dem Ausdruck "zusammenhängend" ist hierin gemeint, dass die Matrix eine Handhabung des Transplantates gestattet, ohne hierbei in Einzelteile oder Bestandteile zu zerfallen. Nicht erforderlich ist, dass alle Bestandteile der Matrix über chemische Bindungen oder Wechselwirkungen aneinander gebunden sind. Eine mechanische Verbindung durch beispielsweise Weben, Walken, Verdrillen oder Ähnliches ist ausreichend.

Mit dem Ausdruck "gerüstbildend" ist hiermit die Eigenschaft der Matrix gemeint, als Strukturbildner für die von den eingewanderten Zellen zu erstellende Gewebematrix zu fungieren. Die Matrix bildet zudem ein Gerüst oder Gatter, in dem sich die Zellen ansiedeln können und Halt finden, um nicht z.B. von Synovialfluid oder Blut aus der Matrix ausgeschwemmt zu werden.

Mit "offener Porosität" ist schließlich im Sinne der Erfindung gemeint, dass die Zwischenräume zwischen den Gerüststrukturen der Matrix einem Stoff- und insbesondere Fluidaustausch mit der Umgebung der Matrix zugänglich sind. Vorzugsweise ist die Porengröße der Poren so bemessen, dass auch ein Eindringen bzw. ein Spülen von Zellen ermöglicht wird. Unter offener Porosität wird im Sinne der Erfindung jedoch auch eine Struktur verstanden, wie sie in Gelen vorliegt. Hier werden durch das Skelett des Gelbildners die Gerüststrukturen der Matrix bereitgestellt. Zwischen diesen befinden sich Hydrathüllen und Fluid, in welche ein Eindringen der Zellen und mit welchen ein Fluidaustausch möglich ist. Entsprechende Gelstrukturen werden daher auch als Matrices mit offener Porosität im Sinne der vorliegenden Erfindung verstanden.

Die Gerüststrukturen mit offener Porosität sind ausgewählt unter Vlies- und Filzstrukturen), Schwämmen, Watte, Wolle, Flechtwerk, geordneten und ungeordneten Faserbündeln, sowie Kombinationen derselben. Bevorzugt weist die Matrix eine Vlies- oder Filzstruktur auf. Kombinationen verschiedener Strukturen beispielsweise in lagenförmiger Anordnung sind möglich und im Bereich der vorliegenden Erfindung.

Das Matrixmaterial kann grundsätzlich jedes geeignete, biologisch und pharmazeutisch annehmbare Material sein. Das Matrixmaterial, das in der erfindungsgemäßen Matrix verwendet wird, kann resorbierbar oder nicht resorbierbar sein. Resorbierbare Materialien sind bevorzugt. Vorzugsweise umfasst die Matrix ein Material, ausgewählt aus der Gruppe, bestehend aus natürlichen und synthetischen Polymeren wie Kollagen, Hyaluronsäure, Chitosan, Chitin, Polysacchariden, Cellulosen und deren Derivaten, Proteinen, Polypeptiden, Polyglycolsäure, Polymilchsäure, Poly(glycolid, lactat), Caprolacton; Keramikmaterialien wie Oxiden, Carbiden, Nitriden und Carbonitriden von Metallen, insbesondere Siliciumoxid, Titanoxid und Calciumoxid; Mineralien wie Halogeniden, insbesondere Fluoriden, Hydroxiden, Phosphaten, Sulfaten von Metallen, vorzugsweise physiologisch unbedenklichen Metallen wie Calciumphosphat, Appatit, Hydroxylappatit; Metallen wie Titan, Aluminium, Gold, Silber, Edelstahl und Gemischen davon. Ganz besonders bevorzugt handelt es sich um Polyglycolsäure (PGA), Polymilchsäure, Kollagen oder Hyaluronsäure.

Als Polyglycolsäuren werden vorzugsweise reine Polyglycolsäuren mit Molekulargewichten von > 20.000, vorzugsweise 30.000 bis 70.000 g/mol, am meisten bevorzugt ca. 50.000 g/mol verwendet. Als Matrixmaterial kann beispielsweise ein Vlies aus Polyglycolsäure, wie es von der Alpha Research Switzerland GmbH unter der Marke PGA-Soft Felt® vertrieben wird, verwendet werden. Bei diesem Produkt beträgt die Resorptionszeit *in vivo* ca. 40 bis 60 Tage. Nach sieben Tagen *in vitro* beträgt die mechanische Festigkeit in Folge der Hydrolyse noch ca. 50 % des Ausgangswertes.

Das zellfreie Transplantat umfasst neben der Matrix zusätzlich ein Gel. Dieses Gel ist auf mindestens einer Seite der Matrix aufgebracht und/oder durchdringt diese mindestens teilweise. Bevorzugt durchdringt das Gel die Matrix vollständig. Vorzugsweise weist die Matrix selbst eine andere Struktur als die eines Gels auf. Ganz besonders bevorzugt sind steifere Strukturen wie sie oben mit Ausnahme der Gele explizit genannt sind. Das Gel hat demnach vorzugsweise eine geringere Steifigkeit als die Matrix. Am meisten bevorzugt sind Vlies- und Filzstrukturen, in die ein Gel eingebracht ist.

Das Gel kann ein natürliches oder synthetisches Hydrogel sein. Es weist vorzugsweise eine geringere Steifigkeit als die Matrix auf. Das Gel kann beispielsweise ausgewählt werden unter Polysacchariden, Polypeptiden, Hyaluronsäure, Fibrin, Kollagen, Alginat, Agarose und Chitosan sowie Salze, Derivate und Mischungen davon. Geeignete Salze sind z.B. Alkali- und Erdalkalisalze der genannten Gele. Am meisten bevorzugt handelt es sich um Hyaluronsäure oder ein Hyaluronsäuresalz wie Na-Hyaluronat.

Als Hyaluronsäurequalitäten können fermentativ hergestellte Qualitäten verwendet werden. Alternativ ist auch die Verwendung von tierisch gewonnener Hyaluronsäure möglich. Das mittlere Molekulargewicht der verwendeten Qualitäten beträgt üblicherweise zwischen 250 und 6.000 kDa, vorzugsweise 1.000 bis 2.000 kDa, am meisten bevorzugt in etwa 1.200 kDa. Geeignete Hyaluronsäureprodukte sind im Handel erhältlich. Geeignet ist beispielsweise die von der TRB Chemedika AG unter der Marke Ostenil® vertriebene Hyaluronsäurequalität. Dieses Material ist CE-zertifiziert und daher für Arzneimittelzwecke geeignet.

Die Gele können durch Quellen, Präzipitation oder Polymerisation eines geeigneten Gelbildners in einer physiologisch geeigneten Lösung gebildet werden. Beispiele für derart geeignete Lösungen sind Wasser sowie wässrige Lösungen von Salzen (z.B. Alkali- und Erdalkalihalogeniden (Cl, Br, I), -carbonaten, -phosphaten, -citraten, -acetaten und Ähnlichem), organischen Säuren, Puffersubstanzen und deren Gemischen. Alternativ können komplexere Lösungen verwendet werden wie Kulturmedium oder Körperfluide oder hiervon abgeleitete Lösungen wie Synovialfluid oder Serum. Die verwendete Menge an Gelbildner ist so bemessen, dass sie eine angemessene Viskosität des Gels bereitstellt. Für Hyaluronsäure liegt diese üblicherweise im Bereich von 0,5-50 mg/ml, vorzugsweise 0,5-20 mg/ml, am meisten bevorzugt 10 mg/ml.

Am meisten bevorzugt ist ein Transplantat aus einem PGA Vlies- oder Filz als Matrix, in die ein Hyaluronsäuregel eingearbeitet ist.

Die Abmessungen des erfindungsgemäßen zellfreien Transplantats richten sich generell nach den Abmessungen des zu behandelnden Defekts bzw. der benötigten Größe des Transplantats. Die Dimensionen sind vom behandelnden Arzt nach Bedarf anzupassen. Für Lesionen in Knorpelgewebe, insbesondere im Kniegelenk liegen diese Größen üblicherweise im Bereich von 10 bis 50 mm Länge, 10 bis 50 mm Breite und 0,5 bis 3 mm Dicke, bevorzugt 10 bis 30 mm Länge, 10 bis 30 mm Breite und 1 bis 2 mm Dicke. Am meisten bevorzugt wären Größen von 20 x 20 mm Breite und Länge und 1,1 bis 2 mm Dicke. Entsprechende Abmessungen sind für nicht quadratische Formen z.B. rechteckig, rund, oval, polyedrisch etc. anpassbar.

Die Kombination von Matrix und Gel im zellfreien Transplantat der vorliegenden Erfindung hat den Vorteil, dass das Gel eine mechanische Barriere gegenüber anderen Zellen als mesenchymalen Vorläuferzellen des durch die Pridie-Bohrung oder ähnliche Frakturen eindringenden Blutes ausbildet. Hierdurch wird eine selektive Einwanderung der mesenchymalen Vorläuferzellen in das Transplantat ermöglicht. Nur diese setzen sich daher in der Matrix fest und differenzieren zu den gewünschten Gewebszellen aus. Eine Überwucherung der erwünschten gewebsbildenden Zellen durch andere Zellen erfolgt damit nicht oder lässt sich wesentlich verringern.

Gleichzeitig fördert das Gel die Retention der gewünschten Zellen unter mechanischer Belastung vor Ausbildung der natürlichen Biomatrix des Knorpels um selbige herum. Dies ermöglicht eine frühere Belastung nach Erhalt des Transplantats durch den Patienten.

Der zweite erforderliche Bestandteil des zellfreien Transplantats gemäß der vorliegenden Erfindung ist Blut-Serum üblicherweise humanes Serum. Dieses kann autolog, allogen oder heterolog sein. Unter Serum wird erfindungsgemäß der flüssige, nach erfolgter Blutgerinnung verbleibende Teil des Blutes verstanden. Serum enthält keine Blutzellen und im Gegensatz zu Blutplasma kein Fibrinogen. Die übrigen Bestandteile des Blutplasmas sind ebenfalls im Serum zu finden. Diese sind Fett, Fettsäuren, Glycerin, Zucker, Salze, Metalle und Plasmaproteine. Die Plasmaproteine umfassen beispielsweise Transportproteine, Enzyme, Proenzyme, Enzyminhibitoren, das Komplementsystem, Immunproteine, Entzündungsmediatoren und Ähnliches.

Das erfindungsgemäß zu verwendende Serum kann durch Zusatz mindestens eines Bestandteils und/oder Entfernung mindestens einer Serumkomponente modifiziert sein. In einer bevorzugten Ausführungsform handelt es sich um nicht modifiziertes Serum. Ganz besonders bevorzugt ist autologes Serum. Dieses kann durch Blutentnahme am Patienten und Gewinnung des Serums mittels herkömmlicher Methoden gewonnen werden. Das so gewonnenen Serum kann dann mit der Matrix und dem Gel, falls vorhanden, in Kontakt gebracht und dadurch in das Transplantat eingearbeitet werden, ggf. direkt am Ort der Transplantation durch den behandelnden Arzt.

Alternativ handelt es sich um modifiziertes Serum. Wenn die Modifikation durch Zusatz mindestens eines Bestandteils erfolgt, kann dieser vorzugsweise ausgewählt sein aus der Gruppe, bestehend aus Wachstumsfaktoren, Differenzierungsfaktoren (siehe hierzu die DE 19957388), Hormonen, Cytokinen, zellulären Adhäsionsmolekülen, chemotaktischen Faktoren einschließlich von Chemokinen wie sie in der WO 2005/014027 beschrieben sind, Enzymen, Enzyminhibitoren, Coenzymen, Mineralien, Fetten, Lipiden, Sacchariden, Arzneimittelwirkstoffen wie Antibiotika, Analgetika, Entzündungshemmern und Immunsuppressiva, Puffersubstanzen, Stabilisatoren insbesondere Kryostabilisatoren, und Vitaminen, vorzugsweise Hormone, Chemokine, Wachstumsfaktoren und Differenzierungsfaktoren. Vorzugsweise sind die Hormone, Chemokine, Wachstums- und Differenzierungsfaktoren ausgewählt unter Insulin, PDGF, IGF, GMCSF, GDF5, GDF6, FGF, BMP2, BMP4, BMP7, IL8, SDF1-α und EGF. Am meisten bevorzugt ist Insulin. Gleichfalls kann die Matrix und/oder das Gel durch Einarbeiten der oben genannten Bestandteile oder Mischungen davon modifiziert werden.

Alternativ können bestimmte Bestandteile wie z.B. Enzyme, Immunproteine, Proenzyme, Zucker etc. vor dem In-Kontakt-Bringen mit der Matrix aus dem Serum entfernt werden. Proteine oder Zucker können z.B. mittels Affinitätschromatographie selektiv entfernt werden. Das Serum kann auch verdünnt werden. Hierzu wird Serum mit der gewünschten Menge an physiologisch annehmbarer Flüssigkeit wie Citratpuffer, PBS oder ähnlichem, gemischt.

Das oben beschriebene zellfreie Transplantat kann hergestellt werden nach einem Verfahren, bei dem man die Matrix und, das Gel mit dem Serum in Kontakt bringt. Dieses In-Kontakt-Bringen kann durch Auftropfen, Einweichen, Imprägnieren oder Tränken erfolgen. Bevorzugt wird, zunächst das Gel in die Matrix eingearbeitet und/oder auf diese aufgebracht, wonach das In-Kontakt-Bringen mit dem Serum erfolgt. Enthält das zellfreie Transplantat weitere Bestandteile, wie oben angegeben, können diese in eines oder mehrere von Matrix, Gel und Serum eingearbeitet werden.

Das erfindungsgemäße Verfahren kann einen Trocknungsschritt enthalten. Die Verwendung eines Trocknungsschrittes hat den Vorteil, dass das Transplantat in trockener Form länger lagerfähig ist. Wird die Matrix und, das Gel vor In-Kontakt-Bringen mit dem Serum getrocknet, kann diese Struktur durch In-Kontakt-Bringen mit Serum, beispielsweise Tränken oder Einweichen rekonstituiert und in einen gebrauchsfertigen Zustand überführt werden. Wird alternativ die Struktur aus Matrix, Gel, und Serum getrocknet, kann durch Tränken oder Einweichen in Serum oder einer anderen, geeigneten pharmazeutisch und physiologisch annehmbaren Lösung, wie oben für die Gelbildung beschrieben, beispielsweise physiologischer Kochsalzlösung rekonstituiert und in einen gebrauchsfertigen Zustand überführt werden. Wenn das erfindungsgemäße Transplantat weitere Bestandteile enthält, können diese auch mit der rekonstituierenden Lösung in das fertige zellfreie Transplantat eingebracht werden. Dies kann insbesondere dann erwünscht sein, wenn es sich bei den weiteren Bestandteilen um Proteine oder labile Cofaktoren handelt.

Für die oben genannten bevorzugten Ausführungsformen aus Polyglycolsäuervlies mit Hyaluronsäuregel werden bei Vliesgrößen von 20 mm x 20 mm x 1,1 mm ca. 400 µl einer Hyaluronsäurelösung (10 mg/ml) in physiologisch geeigneter Lösung oder bereits in Serum in das Material eingebracht. Bei Vliesen von 20 mm x 20 mm x 2 mm werden ca. 730 µl Hyaluronsäurelösung verwendet. Werden Transplantate dieser Dimensionen beispielsweise durch Lyophilisierung getrocknet, können sie durch Tränken mit 1 bis 2 ml Lösung rekonstituiert werden. Bei trockenen Matrices ohne Serum wird vorzugsweise mit Serum oder verdünntem Serum rekonstituiert, bei trockenen Matrices mit Serum vorzugsweise in physiologischer Kochsalzlösung.

Geeignete Serumkonzentrationen sind 1 bis 100 Vol.-% des von der Matrix gehaltenen Volumens an Gel und Fluid. Zur Verringerung der Serumkonzentration unter 100 % kann mit physiologischer Kochsalzlösung verdünntes Serum eingesetzt werden.

Für Matrizes mit Gel werden Serumgehalte von 0,01 bis 50 Vol.-%, stärker bevorzugt 0,5 bis 20 Vol.-% und am meisten bevorzugt 1 bis 10 Vol.-% Serum, bezogen auf das Gesamtvolumen an Gel, Serum und ggf. pharmazeutisch annehmbarer Flüssigkeit, verwendet.

Das erfindungsgemäße zellfreie Transplantat kann zur Gewebsregeneration und insbesondere zur Regeneration von Knorpel und/oder Knochen verwendet werden. Vorzugsweise wird es zur Regeneration mesenchymaler Gewebe verwendet. Am meisten bevorzugt ist die Verwendung zur Knorpel- und/oder Knochenregeneration, insbesondere nach Pridie-Bohrung oder Mikrofrakturierung. Das Transplantat fungiert als intelligente Abdeckung, welche nach einer Pridie-Bohrung oder Mikrofrakturierung zur Wiederherstellung der Gelenkoberfläche in den Knorpel passgenau eingebracht wird. Das Matrixmaterial, vorzugsweise Filzmaterial dient der mechanischen Stabilität und fungiert als Leitstruktur, welche die homogene, dreidimensionale Verteilung von aus dem Knochenmark bzw. spongiösem Knochen einwandernden Patientenzellen fördert. Das Gel wie Hyaluronsäure wirkt als Barriere, um die Einwanderung von roten Blutzellen und Leukozyten zu verhindern. Das Serum, vorzugsweise autologes Serum begünstigt die Einwanderung von Zellen, speziell mesenchymalen Vorläuferzellen in das Transplantat und somit in den Defekt. Das Reifen oder die Ausdifferenzierung der in das Transplantat eingewanderten mesenchymalen Vorläuferzellen zu Chondrozyten und damit der Aufbau eines knorpeligen Regeneratgewebes wird durch die Hyaluronsäure, das Serum und die im Gelenk vorhandene Synovialflüssigkeit induziert. Überraschend hat sich gezeigt, dass die Verwendung von Serum deutlich gesteigerte Rekrutierungszahlen ermöglicht.

Die folgenden Beispiele sollen die vorliegende Erfindung lediglich veranschaulichen, diese jedoch nicht beschränken.

### Beispiel 1

### Rekrutierung humaner mesenchymaler Stammzellen mittels Wachstums- und Differenzierungsfaktoren, Chemokinen und humanem Serum in vitro

### A Isolierung und Kultivierung humaner mesenchymaler Stammzellen

Die Isolierung humaner mesenchymaler Stammzellen (MSC) aus dem Knochenmark ist beschrieben [DE 103 33 901]. Maximal 3 ml Knochenmarkspunktat werden mit 10 ml PBS gemischt und für 10 Min. und 310 g bei Raumtemperatur zentrifugiert. Das Zellpellet wird resuspendiert und erneut mit PBS gewaschen. Die Zellen werden in 20 ml DME-Medium (mit 10-20% FBS, 2% HEPES, 4 mM L-Glutamin, 100 U/ml Penicillin, 100 µg/ml Streptomycin) aufgenommen. Je 5 ml dieser Zellsuspension wird auf 20 ml eines Percoll Dichtegradienten der Dichte 1,073 g/ml gegeben. Die Zellen werden bei 900 g für 32 Min. zentrifugiert. Die obere Phase wird in ein neues Zentrifugenröhrchen überführt. Nach Zugabe des 2,5-fachen Volumens PBS erfolgt erneut das Zentrifugieren bei 310 g für 6 Minuten. Das Zellpellet wird in DME-Medium aufgenommen. 1,5*10⁵-3,5*10⁵ Zellen/cm² werden zur Kultur in eine Zellkulturflasche geben und bei 37 °C, 5% CO₂ inkubiert. Der erste Mediumwechsel erfolgt nach 72 Stunden, dann alle 3-4 Tage. Die so isolierten Zellen wachsen nach 2-3 Wochen konfluent und werden dann mittels Trypsinisieren in einer Zelldichte von 6.000 Zellen/cm² Kulturoberfläche in ein neues Kulturgefäß überführt (Passage 1). Nach circa einer Woche werden die Zellen erneut trypsinisiert (Passage 2). Die Homogenität der vorliegenden Kultur humaner mesenchymaler Stammzellen wird mittels FACS-Analyse verifiziert, wobei die Oberflächenantigene Endoglin und ALCAM nachzuweisen und die Oberflächenantigene CD34, CD 45 und CD 14 nicht nachzuweisen sind.

### B Prüfung der chemotaktischen Aktivität von Wachstums- und Differenzierungsfaktoren (CDMP1, CDMP2) und Chemokinen (SDF1-α, IL8) auf humane mesenchymale Stammzellen in vitro

Die chemotaktische Wirkung von Wachstums- und Differenzierungsfaktoren, wie beispielsweise cartilage derived morhogenetic protein-1 (CDMP1 oder growth and differentiation factor-5, GDF5) und cartilage derived morphogenetic protein-2 (CDMP2 oder growth and differentiation factor-6, GDF6) auf mesenchymale Stamm- und Vorläuferzellen ist beschrieben [DE 199 57 388]. Die chemotaktische Wirkung bzw. die Verwendung von Chemokinen, wie beispielsweise stromal derived factor-1α (SDP1-α) oder Interleukin-8 (IL8), zur Rekrutierung von mesenchymalen Stamm- und Vorläuferzellen ist ebenfalls beschrieben [DE 103 33 901].

Die Prüfung der chemotaktischen Aktivität erfolgt in sogenannten 96-Loch Chemotaxis-Platten (ChemoTx system, Neuroprobe, USA). Das Testprinzip sieht vor, dass die zu prüfende Substanz in Lösung definierter Menge und Konzentration in eine Vertiefung (Well) gegeben wird. Die Vertiefung wird daraufhin von einer mit Poren versehenen Membran (Porengröße hier 8 µm) abgedeckt, so dass die Membranunterseite von der die chemotaktische Substanz enthaltenden Lösung benetzt wird.

Auf die Membranoberseite wird gegenüber der Vertiefung eine definierte Menge an Zellsuspension gegeben, welche die zu prüfende Substanz nicht enthält. Nach einigen Stunden bildet sich von der unteren Vertiefung ausgehend über die Membran hinweg zur Zellsuspension ein Konzentrationsgradient an zu prüfender Substanz aus. Ist die zu prüfende Substanz chemotaktisch aktiv, wandern Zellen der Zellsuspension durch die Poren der Membran zur Membranunterseite bzw. in die untere Vertiefung. Die gewanderten Zellen werden angefärbt und ihre Anzahl mit Hilfe eines Mikroskops bestimmt.

Zur Kontrolle werden untere Vertiefungen mit dem Lösungsmittel der zu prüfenden Substanz versehen, mit der Membran bedeckt und mit der Zellsuspension überschichtet. Durch mikroskopische Zählung der Zellen auf der Membranunterseite (Fläche: 25 mm²) bzw. in der unteren Vertiefung erfolgt die Bestimmung der spontan, ohne Stimulus durch die chemotaktische Substanz gewanderten Zellen. Zur Ermittlung der Zellzahl, welche durch die chemotaktische Substanz rekrutiert wurde, wird die Anzahl der spontan gewanderten Zellen abgezogen.

Vor Testbeginn der oben genannten Wachstums- und Differenzierungsfaktoren und der Chemokine wurden humane mesenchymale Stammzellen des Knochenmarks 24 Stunden lang mit Diätmedium (DME-Medium + 1% Penicillin/Streptomycin + 0,5% bovines Serumalbumin, BSA) versetzt. Die Wachstums- und Differenzierungsfaktoren und die Chemokine wurden in verschiedenen Mengen in Diätmedium aufgenommen, so dass definierte Lösungen von jeweils 250 nM, 500 nM, 750 nM und 1000 nM der Faktoren vorlagen. 36 µl der jeweiligen Lösungen wurden im Triplikat in untere Vertiefungen der 96-Loch Chemotaxis-Platte gegeben und mit der Membran bedeckt, so dass die Membranunterseite von den Lösungen benetzt war. Als Kontrolle diente Diätmedium.

Auf die Membranoberseite wurden 40 µl Diätmedium mit 30.000 humanen mesenchymalen Stammzellen gegeben. Nach 20 Stunden Kultivierung im Brutschrank bei 37°C, 5% CO₂ wurde die Membran entfernt und zur Fixierung der Zellen für 3 Minuten in eiskaltes Ethanol/Aceton (1:1 v/v) gegeben. Die Membranoberseite wurde mittels eines Wattestäbchen gründlich von anhaftenden Zellen gesäubert. Zellen auf der Membranunterseite wurden mittels Hemacolor-Lösung (Merck, Darmstadt) gefärbt. In der unteren Vertiefung konnten keine Zellen detektiert werden.

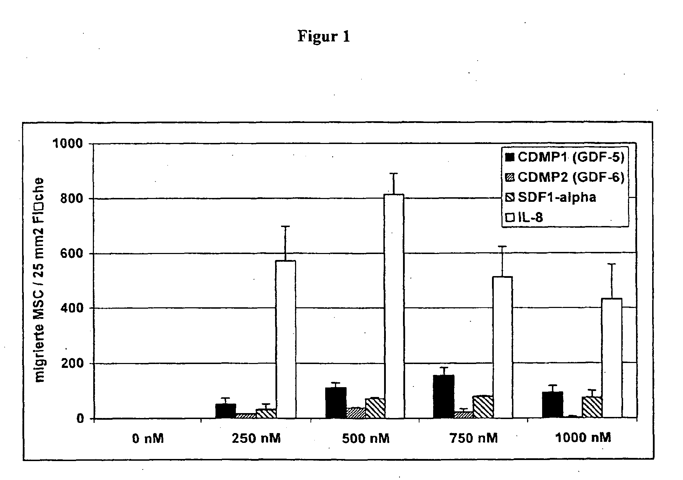

Die Zählung der auf der Membranunterseite befindlichen Zellen erfolgte durch Auszählen am Mikroskop. Die Anzahl der durch CDMP1, CDMP2, SDF1-α und IL8 in verschiedenen Konzentrationen rekrutierten Stammzellen wurden nach Subtraktion der gewanderten Zellen im Kontrollansatz (ohne chemotaktischen Faktor) bestimmt. Die Mittelwerte der Zellzahlen mit Standardabweichung sind für die jeweiligen Faktoren in der Fig. 1 dargestellt. Durch CDMP1 konnten maximal 156 MSC (750 nM CDMP1) pro 25 mm² zur Wanderung angeregt werden. CDMP2 rekrutierte maximal 38 MSC (500 nM CDMP2) pro 25 mm², SDF1-α maximal 79 MSC (750 nM SDF1-α) pro 25 mm² und IL8 maximal 814 MSC (500 nM IL8) pro 25 mm².

### C Prüfung der chemotaktischen Aktivität von humanem Serum des Blutes auf humane mesenchymale Stammzellen in vitro

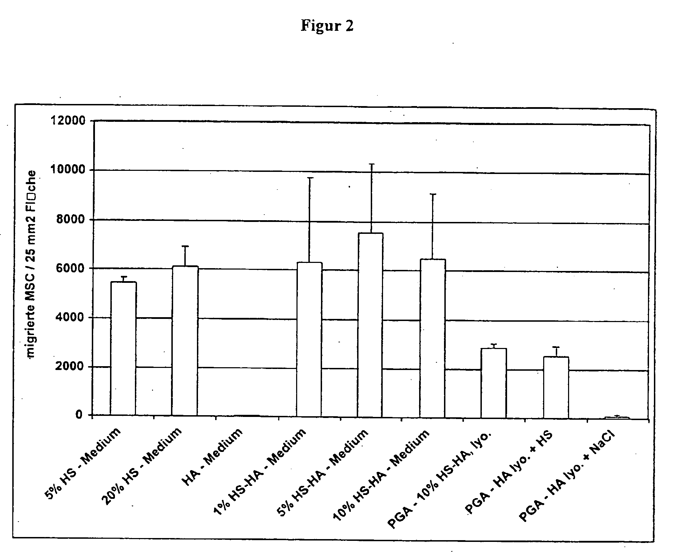

Die Testung von humanem Serum mittels des in B beschriebenen Testverfahrens ergab überraschenderweise, dass humanes Serum im Vergleich zu Wachstum- und Differenzierungsfaktoren und Chemokinen eine deutlich stärkere chemotaktische Wirkung auf humane mesenchymale Stamm- und Vorläuferzellen *in vitro* ausübt. Die Zellzahlen der durch humanes Serum in verschiedenen Formulierungen (in Diätmedium oder in Hyaluronsäure) rekrutierten humanen mesenchymalen Stamm- und Vorläuferzellen im Mittel mit entsprechenden Standardabweichungen sind in der Fig. 2 dargestellt. Je nach Formulierung konnten durch humanes Serum minimal 2.135 (PGA - HA lyo. + HS) und maximal 10.332 (5% HS-HA - Medium) humane mesenchymale Stamm- und Vorläuferzellen rekrutiert werden. Die Prüfung von Hyaluronsäure ohne humanes Serum in Diätmedium als chemotaktischer Faktor ergab im Mittel 24 (HA-Medium) bzw. im Mittel 48 (PGA - Ha lyo. + NaCl) humane mesenchymale Stamm- und Vorläuferzellen, welche rekrutiert wurden.

Folgende verschiedenen Formulierungen von humanem Serum wurden im beschriebenen Testverfahren zur Rekrutierung von humanen mesenchymalen Stamm- und Vorläuferzellen eingesetzt. Humanes Serum wurde aus Vollblutproben (n=5) ohne Antikoagulanzien nach natürlicher Gerinnung gewonnen, zu gleichen Teilen gemischt und zur Herstellung der verschiedenen Serumformulierungen eingesetzt. Zur Herstellung der Ansätze "5% HS - Medium" und "10% HS - Medium" wurde Diätmedium mit humanem Serum versetzt, so dass 5%ige bzw. 10%ige Lösungen resultierten. Der Ansatz "HA - Medium" besteht aus Diätmedium und fermentativ gewonnener Hyaluronsäure (Ostenil®, TRB Chemedica AG) mit einem mittleren Molekulargewicht von 1.200 kDa zu gleichen Teilen. Die Ansätze "1% - HS-HA - Medium", "5% - HS-HA - Medium" und "10% - HS-HA - Medium" bestehen aus "HA - Medium", welchem humanes Serum zugesetzt wurde, so dass 1%ige, 5%ige bzw. 10%ige Lösungen resultierten.

Zur Herstellung des Ansatzes "PGA - 10% HS-HA, lyo." wurden 270 µl fermentativ gewonnene Hyaluronsäure (Ostenil®, TRB Chemedica AG) mit 30µl humanem Serum gemischt und in ein aus Polyglykolsäure (PGA) bestehendes Vlies (PGA-Soft Felt®, Alpha Research Switzerland GmbH) mit den Abmessungen 20mm x 15mm x 1,1mm eingebracht. Das mit dem Hyaluronsäure-Serum-Gemisch getränkte Vlies wurde für 1 Stunde bei - 20°C gefroren und danach für 16 Stunden im Lyophilisator gefriergetrocknet. Zum Rekonstituieren wurde das gefriergetrocknete Vlies für 10 Minuten in 1 ml physiologische Kochsalzlösung gegeben, um anschließend mittels Zentrifugation für 10 Minuten bei 2.000 rpm zirka 80-100 µl der Hyaluronsäure-Serum Lösung zu gewinnen. Durch Verdünnen der Lösung mit Diätmedium zu gleichen Teilen wurde die Formulierung "PGA - 10% HS-HA, lyo." hergestellt und direkt zur Prüfung der chemotaktischen Aktivität eingesetzt.

Zur Herstellung des Ansatzes "PGA - HA, lyo. + HS" wurden 300 µl fermentativ gewonnener Hyaluronsäure (Ostenil®, TRB Chemedica AG) in ein aus Polyglykolsäure (PGA) bestehendes Vlies (PGA-Soft Felt®, Alpha Research Switzerland GmbH) mit den Abmessungen 20mm x 15mm x 1,1mm eingebracht. Das mit der Hyaluronsäure getränkte Vlies wurde für 1 Stunde bei -20°C gefroren und danach für 16 Stunden im Lyophilisator gefriergetrocknet. Zum Rekonstituieren wurde das gefriergetrocknete Vlies für 10 Minuten in 1 ml humanes Serum gegeben, um anschließend mittels Zentrifugation für 10 Minuten bei 2.000 rpm zirka 80-100 µl der Hyaluronsäure-Serum Lösung zu gewinnen. Durch Verdünnen der Lösung mit Diätmedium zu gleichen Teilen wurde die Formulierung "PGA - HA, lyo. + HS" hergestellt und direkt zur Prüfung der chemotaktischen Aktivität eingesetzt.

Zur Herstellung des Ansatzes "PGA - HA, lyo. + NaCl" wurden 300 µl fermentativ gewonnener Hyaluronsäure (Ostenil®, TRB Chemedica AG) in ein aus Polyglykolsäure (PGA) bestehendes Vlies (PGA-Soft Felt®, Alpha Research Switzerland GmbH) mit den Abmessungen 20mm x 15mm x 1,1 mm eingebracht. Das mit der Hyaluronsäure getränkte Vlies wurde für 1 Stunde bei -20°C gefroren und danach für 16 Stunden im Lyophilisator gefriergetrocknet. Zum Rekonstituieren wurde das gefriergetrocknete Vlies für 10 Minuten in 1 ml physiologische Kochsalzlösung gegeben, um anschließend mittels Zentrifugation für 10 Minuten bei 2.000 rpm zirka 80-100 µl der Hyaluronsäure Lösung zu gewinnen. Durch Verdünnen der Lösung mit Diätmedium zu gleichen Teilen wurde die Formulierung "PGA - HA, lyo. + NaCl" hergestellt und direkt zur Prüfung der chemotaktischen Aktivität eingesetzt.

### Ausführungsbeispiel 2

Ein kommerziell im Handel unter der Marke PDA-Soft Felt® von der Alpha Research Switzerland GmbH vertriebenes Polyglycolsäurevlies wurde auf die Abmessungen von 20 mm x 15 mm x 1,1 mm geschnitten. Das Material wurde wie in Beispiel 1 mit einem 10 % Serum enthaltenden Hyaluronsäuregemisch getränkt und anschließend getrocknet. Die Trocknung erfolgte zunächst bei -20°C und anschließend für 16 Stunden im Lyophilisator. Das Vlies wurde durch Tränken mit 1 bis 2 ml physiologischer Kochsalzlösung für 10 min rekonstituiert.

In einem alternativen Ansatz wurde das Vlies mit reiner Hyaluronsäure, 10 mg/ml gelöst in physiologischer Kochsalzlösung, getränkt. Das so erstellte Material wurde wie oben getrocknet. Die Rekonstitütion erfolgte durch Tränken in 1 bis 2 ml Serum für 10 min. Beide Vliese können unmittelbar für die Transplantation verwendet werden.

## Patentansprüche

1. Zellfreies Transplantat, umfassend (i) eine zusammenhängende, gerüstbildende Matrix mit offener Porosität aus einem biologisch und pharmazeutisch annehmbaren Material ausgewählt aus Vlies-oder Filzstrukturen Schwämmen, Watte, Wolle, Flechtwerk, geordneten und ungeordneten Faserbündeln, sowie Kombinationen derselben; und (ii) Blutserum,
wobei das zellefreie Transplantat zusätzlich ein Gel aufweist, das auf mindestens einer Seite der Matrix aufgebracht ist und/oder diese mindestens teilweise durchdringt.

2. Zellfreies Transplantat nach Anspruch 1, worin das Matrixmaterial resorbierbar oder nicht resorbierbar ist.

3. Zellfreies Transplantat nach einem der vorstehenden Ansprüche, worin die Matrix ein Material umfasst, ausgewählt aus der Gruppe, bestehend aus natürlichen und synthetischen Polymeren wie Kollagen, Hyaluronsäure, Chitosan, Chitin, Polysacchariden, Cellulosen und deren Derivate, Proteinen, Polypeptiden, Polyglycolsäure, Polymilchsäure, Poly(glycolid,lactat), Caprolacton; Keramikmaterialien wie Oxiden, Carbiden, Nitriden und Carbonitriden von Metallen; Mineralien wie Halogeniden, Hydroxiden, Phosphaten, Sulfaten von Metallen wie Calciumphosphat, Appatit, Hydroxylappatit; Metallen wie Titan, Aluminium, Gold, Silber, Edelstahl und Gemischen davon.

4. Zellfreies Transplantat nach Anspruch 1, worin das Gel ein natürliches oder synthetisches Hydrogel ist.

5. Zellfreies Transplantat nach Anspruch 1 oder 4, worin das Gel eine geringere Steifigkeit als die Matrix aufweist.

6. Zellfreies Transplantat nach einem der Ansprüche 1, 4 bis 5, worin das Gel ausgewählt ist unter Polysacchariden, Polypeptiden, Hyaluronsäure, Fibrin, Kollagen, Alginat, Agarose und Chitosan sowie Mischungen davon.

7. Zellfreies Transplantat nach einem der vorstehenden Ansprüche, worin das Blutserum autolog, allogen oder heterolog ist und ggf. durch Zusatz mindestens eines Bestandteils und/oder Entfernung mindestens einer Serumkomponente modifiziert ist.

8. Zellfreies Transplantat nach einem der vorstehenden Ansprüche, zusätzlich enthaltend einer oder mehrere Bestandteile, ausgewählt aus der Gruppe, bestehend aus Wachstumsfaktoren, Differenzierungsfaktoren, Hormonen, Chemokinen, Cytokinen, zellulären Adhäsionsmolekülen, chemotaktischen Faktoren, Enzymen, Enzyminhibitoren, Coenzymen, Mineralien, Fetten, Lipiden, Sacchariden, Arzneimittelwirkstoffen, Puffersubstanzen, Stabilisatoren, und Vitaminen.

9. Zellfreies Transplantat nach Anspruch 8, worin die Hormone, Wachstums- und Differenzierungsfaktoren Insulin, PDGF, IGF, GMCSF, GDF5, GDF6, FGF, BMP2, BMP4, BMP7, IL8, SDF1-α und EGF und Kombinationen davon umfassen.

10. Verfahren zur Herstellung eines zellfreien Transplantats nach einem der vorstehenden Ansprüche, bei dem man die Matrix und das Gel mit dem Blutserum, in Kontakt bringt.

11. Verfahren nach Anspruch 10, worin das In-Kontakt-bringen durch Auftropfen, Einweichen, Imprägnieren oder Tränken erfolgt.

12. Verfahren nach Anspruch 10 oder 11, worin zunächst das Gel in die Matrix eingearbeitet und/oder auf diese aufgebracht wird, und dann das In-Kontakt-bringen mit dem Blutserum erfolgt.

13. Verfahren nach einem der Ansprüche 10-12, worin die Kombination aus Gel und Matrix sowie ggf. weiteren Bestandteilen vor oder nach dem In-Kontakt-bringen getrocknet wird.

14. Verfahren nach Anspruch 13, worin das Transplantat aus dem trockenen Zustand rekonstituiert wird.

15. Zellfreies Transplantat nach einem der Ansprüche 1 bis 9 zur Verwendung zur Gewebsregeneration.

16. Zellfreies Transplantat nach Anspruch 15 zur Verwendung zur Regeneration mesenchymaler Gewebe, insbesondere Knorpel und/oder Knochen.

## Claims

1. A cell-free graft, comprising (i) a continuous skeletal-forming matrix having open porosity of a biologically and pharmaceutically acceptable material, selected from non-woven or felt structures, sponges, wadding, wool, wattle, arranged and not arranged fiber bundles, as well as combinations thereof, and (ii) blood serum, wherein the cell-free graft in addition comprises a gel being applied on at least one side of the matrix and/or at least partially permeating the same.

2. The cell-free graft according to claim 1, wherein the matrix material is absorbable or is non-absorbable.

3. The cell-free graft according to one of the preceding claims, wherein the matrix comprises a material which is selected from the group consisting of natural and synthetic polymers such as collagen, hyaluronic acid, chitosan, chitin, polysaccharides, celluloses and their derivatives, proteins, polypeptides, polyglycolic acid, polylactic acid, poly(glycolide,lactate), caprolactone; ceramic materials such as oxides, carbides, nitrides and carbonitrides of metals; minerals such as halides, hydroxides, phosphates, sulfates of metals such as calcium phosphate, apatite, hydroxyapatite; metals such as titanium, aluminium, gold, silver, stainless steel and mixtures thereof.

4. The cell-free graft according to claim 1, wherein the gel is a natural or synthetic hydrogel.

5. The cell-free graft according to claim 1 or 4, wherein the gel has a lower rigidity than the matrix.

6. The cell-free graft according to one of claims 1, 4 to 5, wherein the gel is selected from polysaccharides, polypeptides, hyaluronic acid, fibrin, collagen, alginate, agarose and chitosan as well as mixtures thereof.

7. The cell-free graft according to one of the preceding claims, wherein the blood serum is an autologous, allogenic or heterologous one and optionally is modified by the addition of at least one ingredient and/or the removal of at least one serum component.

8. The cell-free graft according to one of the preceding claims, additionally containing one or more ingredients which are selected from the group consisting of growth factors, differentiation factors, hormones, chemokines, cytokines, cellular adhesion molecules, chemotactic factors, enzymes, enzyme inhibitors, coenzymes, minerals, fats, lipids, saccharides, drugs, buffering agents, stabilizers and vitamins.

9. The cell-free graft according to claim 8, wherein the hormones, growth and differentiation factors comprise insulin, PDGF, IGF, GMCSF, GDF5, GDF6, FGF, BMP2, BMP4, BMP7, IL8, SDF1-α and EGF and combinations thereof.

10. A method for the production of a cell-free graft according to one of the preceding claims, wherein the matrix and the gel are brought into contact with the blood serum.

11. The method according to claim 10, wherein the contacting step is carried out by dropping, soaking, impregnating or steeping.

12. The method according to claim 10 or 11, wherein at first the gel is worked into the matrix and/or is applied onto the same and then the step of contacting with the blood serum is carried out.

13. The method according to one of claims 10-12, wherein the combination of gel and matrix as well as optionally further ingredients is dried before or after the contacting step.

14. The method according to claim 13, wherein the graft is reconstituted from the dry state.

15. The cell-free graft according to one of claims 1 to 9 for use for tissue regeneration.

16. The cell-free graft according to claim 15 for use for regeneration of mesenchymal tissues, in particular cartilage and/or bones.

## Revendications

1. Greffon acellulaire, qui comprend (i) une matrice continue, formant une structure à pores ouverts, à partir d'une matière acceptable au point de vue biologique et pharmaceutique, choisie parmi des structures de non-tissés ou de feutre, des éponges, de la ouate, de la laine, des nattés, des fibres ordonnés ou désordonnés, ainsi que des combinaisons de ceux-ci, et (ii) du sérum sanguin,
sachant que le greffon acellulaire comprend en supplément un gel, qui est appliqué sur au moins une face de la matrice et / ou est diffusé au moins partiellement dans celle-ci.

2. Greffon acellulaire selon la revendication 1, sachant que la matière de la matrice est résorbable ou non résorbable.

3. Greffon acellulaire selon l'une des revendications précédentes, sachant que la matrice comprend une matière, qui est choisie à partir du groupe comprenant des polymères naturels et synthétiques, comme collagènes, acide hyaluronique, chitosan, chitine, polysaccharides, celluloses et leurs dérivés, protéines, polypeptides, acide de polyglycol, acide polylactique, poly(glycolid, lactate), caprolacton ;
des matières céramiques, comme oxydes, carbures nitrures et carbonitrures de métaux ;
des minéraux, comme halogénures, hydroxydes, phosphates, sulfates de métaux, comme phosphate de calcium, apatite, apatite hydroxylique ;
des métaux, comme titan, aluminium, or, argent, acier inoxydable et mélanges de ceux-ci.

4. Greffon acellulaire selon la revendication 1, dans lequel le gel est un hydrogel naturel ou synthétique.

5. Greffon acellulaire selon revendication 1 ou 4, dans lequel le gel présente une rigidité plus faible que la matrice.

6. Greffon acellulaire selon l'une des revendications 1, 4 à 5, dans lequel le gel est choisi parmi les polysaccharides, polypeptides, acide hyaluronique, fibrine, collagènes, alginate, agarose et chitosan, ainsi que des mélanges de ceux-ci.

7. Greffon acellulaire selon l'une des revendications précédentes, dans lequel le sérum sanguin est autologue, allogène ou hétérologue et, le cas échéant, est modifié par adduction d'au moins un composant et / ou l'élimination d'au moins un composant du sérum.

8. Greffon acellulaire selon l'une des revendications précédentes, qui comprend en supplément un ou plusieurs composants, choisis dans le groupe comprenant des facteurs de croissance, des facteurs de différenciation, des hormones, des chimiokines, des cytokines, des molécules d'adhésion cellulaires, des facteurs chimiotactiques, des enzymes, des inhibiteurs d'enzymes, ce coenzymes, des minéraux, des graisses, des lipides, des saccharines, des substances actives pharmaceutiques, des substances tampons, des stabilisants et des vitamines.

9. Greffon acellulaire selon la revendication 8, dans lequel les hormones, les facteurs de croissance et de différenciation comprennent : insuline, PDGF, IGF, GMCSF, GDF5, GDF6, FGF, BMP2, BMP4, BMP7, IL8, SDF1-α et EGF, ainsi que des combinaisons de ceux-ci.

10. Procédé de fabrication d'un greffon acellulaire selon l'une des revendications précédentes, dans lequel on met la matrice et le gel en contact avec le sérum sanguin.

11. Procédé selon la revendication 10, dans lequel la mise en contact est effectuée par application en gouttes, trempage, imprégnation ou imbibition.

12. Procédé selon revendication 10 ou 11, dans lequel le gel est tout d'abord incorporé dans la matrice et / ou appliqué sur celle-ci, et la mise en contact avec le sérum sanguin est effectuée ensuite.

13. Procédé selon l'une des revendications 10 à 12, dans lequel la combinaison gel et matrice, ainsi que, le cas échéant, d'autres composants sont séchés, avant ou après la mise en contact.

14. Procédé selon la revendication 13, dans lequel le greffon est reconstitué à partir de l'état sec.

15. Greffon acellulaire selon l'une des revendications 1 à 9, destiné à être utilisé pour la régénération de tissus.

16. Greffon acellulaire selon la revendication 15, destiné à être utilisé pour la régénération de tissus mésenchymes, en particulier de cartilages et / ou d'os.
